# EUROPÄISCHE PATENTSCHRIFT

(11) **EP 2 692 301 B1**
(45) Veröffentlichungstag und Bekanntmachung des Hinweises auf die Patenterteilung: **11.03.2015**
(21) Anmeldenummer: 13178900.0
(22) Anmeldetag: 01.08.2013
(51) Int. Cl.: A61B 17/32, A61B 17/52, A61B 17/16

(54) **Medizinisches Werkzeug**
Medical tool
Outil médical

(30) Priorität: 03.08.2012 DE 102012107147
(43) Veröffentlichungstag der Anmeldung: 05.02.2014
(73) Patentinhaber: Karl Storz GmbH & Co. KG, 78532 Tuttlingen (DE)
(72) Erfinder: Berberich, Sascha, 78532 Tuttlingen (DE)

(56) Entgegenhaltungen:
- EP-A1- 1 797 824
- WO-A2-2008/011308
- CN-Y- 201 164 500
- DE-A1-102006 034 756
- US-A- 2 436 538

## Beschreibung

Die vorliegende Erfindung ist auf ein medizinisches Werkzeug zum Abtragen von Gewebe und auf ein medizinisches Instrument mit einem derartigen Werkzeug.

Die vorliegende Erfindung ist insbesondere auf das Abtragen von Knochengewebe oder anderem harten Gewebe bezogen, bei dem Abrieb am Werkzeug entstehen kann.

In DE 10 2006 034 756 A1 und in DE 10 2009 010 561 A1 ist jeweils ein medizinisches Instrument zum Schneiden von Gewebe beschrieben. In einem rohrförmigen Außenschaft rotiert ein rohrförmiger Innenschaft. Außenschaft und Innenschaft weisen jeweils Fenster mit Schneiden auf. Gewebe, das durch Zusammenwirkung der Schneiden am Außenschaft und am Innenschaft abgetrennt wird, wird durch den Innenschaft abgesaugt.

Vor allem beim Abtragen von Knochengewebe oder anderem harten und/oder zähen Gewebe übt medizinisches Personal oft große Kräfte und Drehmomente auf das proximale Ende des medizinischen Instruments aus, um das distale Ende beispielsweise mit möglichst großer Kraft gegen eine zu bearbeitende Gewebeoberfläche zu drücken. Diese Kräfte und Momente können das medizinische Instrument (in der Regel elastisch) verformen, insbesondere einen Schaft des medizinischen Instruments biegen.

Die beschriebene Verformung des medizinischen Instruments und/oder andere Gründe können bewirken, dass der Innenschaft gegenüber seiner vorgesehenen Position relativ zum Außenschaft verschoben oder gekippt wird. Dadurch können der Innenschaft und der Außenschaft einander in einer nicht vorgesehenen Weise berühren. Berührungen des Innenschafts und des Außenschafts, insbesondere der Schneiden am Innen- und Außenschaft, können den Verschleiß an beiden erhöhen und Abrieb in Form feiner Partikel oder sogar den Ausbruch größerer Partikel bewirken.

Auf die beschriebene Weise oder auf andere Weise erzeugte Partikel sind Fremdkörper, die nicht am Operationsort zurückbleiben sollten. Die Partikel werden beispielsweise mit oder ohne Verwendung einer Spülflüssigkeit abgesaugt oder abgeschabt. Trotzdem im Operationsgebiet zurückbleibende Partikel weisen zwar bei geeigneter Wahl der Materialien des medizinischen Instruments keine toxische Wirkung auf. Die Partikel können aber mechanisch nachteilig wirken und beispielsweise bei nachfolgenden Röntgenaufnahmen Artefakte bilden. Abrieb an medizinischen Instrumenten, Vermeidung und/oder Entfernung des Abriebs sind deshalb weiterhin aktuelle Herausforderungen.

Eine Aufgabe der vorliegenden Erfindung besteht darin, ein verbessertes medizinisches Werkzeug und ein verbessertes medizinisches Instrument zum Abtragen von Gewebe zu schaffen.

Diese Aufgabe wird durch die Gegenstände der unabhängigen Ansprüche gelöst. Weiterbildungen sind in den abhängigen Ansprüchen angegeben.

Herkömmlich wird versucht, durch geeignete Materialien und geeignete Formgebung Verschleiß und Abrieb zu minimieren. Ferner wird versucht, ein unvermeidliches Minimum an Abrieb durch Absaugen während des Abtragens von Gewebe oder bei einem nachfolgenden Verfahrensschritt zu entfernen. Demgegenüber beruhen Ausführungsformen der vorliegenden Erfindung auf der Idee, durch Abrieb oder auf andere Weise entstandene Partikel mittels eines am medizinischen Werkzeug selbst angeordneten Magneten zu sammeln.

Während herkömmlich medizinische Instrumente in der Regel überwiegend oder ausschließlich aus nicht-magnetisch und nicht-magnetisierbaren Materialien (insbesondere rostfreien Stählen) bestehen, bedingt die vorliegende Erfindung eine Verwendung magnetischer oder magnetisierbarer Materialien zumindest im Bereich von Oberflächen, die durch Abrieb oder Abtrag betroffen sein können.

Die Offenlegungsschrift DE 10 2006 034 756 A1 offenbart den Gegenstand der Präambel von Anspruch 1.

In EP 0 576 306 B1, auch veröffentlicht als DE 693 17 699 T2, ist ein magnetischer Detektor beschrieben, der für eine Anordnung in einer Fluidströmung vorgesehen ist. Nach Aufnahme einer vorbestimmten Menge metallischer Partikel soll ein Alarm ausgelöst werden.

In US 4,810,148 und EP 0 882 510 A1 sind Schrauben mit Magneten beschrieben, die dafür vorgesehen sind, von zirkulierendem Schmiermittel umspült zu werden. Im zirkulierenden Schmiermittel enthaltener metallischer Abrieb soll an den Magneten haften bleiben. In WO 02/081859 A1, auch veröffentlicht als EP 1 373 676 A1 und DE 601 22 936 T2, ist eine Vorrichtung zum Wiedergewinnen von metallischem Abrieb aus einem Bohrloch beschrieben, die eine Mehrzahl von Magneten aufweist.

Keine der gefundenen Druckschriften weist einen Bezug zur Medizintechnik auf oder lässt irgendeine Anwendbarkeit auf medizinische Aufgabenstellungen erkennen. Die drei erstgenannten Druckschriften, EP 0 576 306 B1, US 4,810,148 und EP 0 882 510 A1 haben überdies keinen Bezug auf irgendeine Bearbeitung einer Oberfläche. Überdies sind sie vorgesehen, um - ganz anders als bei der vorliegenden Erfindung - von einem Medium, das metallischen Abrieb enthält, umspült zu werden. Die letztgenannte Druckschrift WO 02/081859 A1 steht zwar in Bezug zu einer spanenden Bearbeitung, wenn auch ebenfalls ohne medizinischen Kontext, sieht jedoch eine separate Vorrichtung zum Sammeln metallischen Abriebs vor. Eine Kombination mit einem Bearbeitungswerkzeug selbst ist weder beschrieben noch auch nur angedeutet oder für Fachleute ausgehend von WO 02/081859 A1 naheliegend.

Magnetisch oder magnetisierbar im Sinne der vorliegenden Erfindung ist jedes Material, das nicht nur diamagnetische Eigenschaften aufweist. Magnetisch oder magnetisierbar sind insbesondere paramagnetische, ferromagnetische, antiferromagnetische und ferrimagnetische Materialien. Anders ausgedrückt werden hier alle Materialien als magnetisch oder magnetisierbar bezeichnet, auf die in einem inhomogenen Magnetfeld eine Kraft in Richtung zu einem Ort mit einer höheren magnetischen Feldstärke wirkt. Körper, die ein derartiges magnetisches oder magnetisierbares Material aufweisen, werden beispielsweise von einem Pol eines Dipolmagneten angezogen. Diamagnetische Materialien erfahren in einem inhomogenen Magnetfeld hingegen eine Kraft in Richtung zu einem Ort mit einer geringeren magnetischen Feldstärke. Ein Körper aus einem diamagnetischen Material wird deshalb von einem Pol eines Dipolmagneten abgestoßen. Ein Material, das lediglich diamagnetische Eigenschaften aufweist, ist hier nicht als magnetisch oder magnetisierbar bezeichnet.

Ein medizinisches Werkzeug zum Abtragen von Gewebe umfasst eine Abtrageinrichtung zum spanenden Abtragen von Gewebe und einen Magneten zum magnetischen Anziehen und Sammeln von magnetischen oder magnetisierbaren Partikeln.

Das medizinische Werkzeug ist insbesondere eine Fräseinrichtung oder ein Shaver. Das medizinische Werkzeug ist für eine Verwendung an oder mit einem medizinischen Instrument vorgesehen und ausgebildet, mit dem es zerstörungsfrei lösbar verbunden werden kann. Alternativ kann das medizinische Werkzeug dauerhafter Bestandteil eines medizinischen Instruments bzw. von dem medizinischen Instrument nicht zerstörungsfrei lösbar sein. Das medizinische Werkzeug ist insbesondere für mikroinvasive Verfahren vorgesehen und ausgebildet.

Die Abtrageinrichtung ist insbesondere am distalen Ende oder nahe dem distalen Ende des medizinischen Werkzeugs angeordnet und insbesondere zum Abtragen von Knochen- oder Knorpelgewebe oder anderem festen, harten und/oder zähen Gewebe ausgebildet. Die Abtrageinrichtung kann eine oder mehrere geometrisch bestimmte Schneiden aufweisen, beispielsweise in der Art eines Fräsers oder eines Bohrers. Alternativ kann die Abtrageinrichtung geometrisch unbestimmte Schneiden aufweisen. Der Magnet ist insbesondere zum magnetischen Anziehen und Sammeln von Partikeln vorgesehen und ausgebildet, die durch Abrieb, Ausbruch oder auf andere Weise durch Verschleiß an der Abtrageinrichtung entstehen.

Ein magnetisches Anziehen und Sammeln von magnetischen oder magnetisierbaren Partikeln kann ein (potentiell traumatisierendes oder zusätzlich traumatisierendes) Abschaben von Oberflächen in vielen Fällen teilweise oder vollständig ersetzen. Der Magnet kann dabei ohne Weiteres und abhängig von seinen eigenen Eigenschaften und den magnetischen Eigenschaften der Partikel die Partikel auch dann anziehen und sammeln, wenn sie beispielsweise in eine weiche Oberfläche teilweise oder vollständig eingedrückt sind. Der Magnet ermöglicht eine selektive Wechselwirkung mit magnetischen oder magnetisierbaren Partikeln, ohne dabei umliegendes Gewebe zu beeinflussen. Das beschriebene medizinische Werkzeug ist somit geeignet, die Traumatisierung zu reduzieren.

Die Integration der Abtrageinrichtung und des Magneten in einem einzigen medizinischen Werkzeug kann ein Sammeln und Entfernen magnetischer oder magnetisierbarer Partikel gleichzeitig mit dem Abtragen von Gewebe oder in einem unmittelbar nachfolgenden separaten Verfahrensschritt mit dem gleichen medizinischen Werkzeug ermöglichen. Die Verwendung des medizinischen Werkzeugs ist deshalb hinsichtlich des Zeitaufwands und damit auch medizinisch und ökonomisch sinnvoll.

Bei einem medizinischen Werkzeug, wie es hier beschrieben ist, ist der Magnet insbesondere an einem während eines Abtragens von Gewebe im Wesentlichen ruhenden Bauteil des Werkzeugs angeordnet.

Der Magnet ist insbesondere an einem während des Abtragens von Gewebe nur linear und insbesondere nur langsam linear bewegten und nicht rotierenden Bauteil des Werkzeugs angeordnet. Das während des Abtragens von Gewebe nicht rotierende oder im Wesentlichen ruhende Bauteil umfasst insbesondere einen Außenschaft oder ein distales Ende eines Außenschafts des medizinischen Werkzeugs.

Bei einem medizinischen Werkzeug, wie es hier beschrieben ist, umfasst die Abtrageinrichtung insbesondere ein erstes Bauteil und ein zweites Bauteil, wobei zum Abtragen von Gewebe das zweite Bauteil relativ zu dem ersten Bauteil bewegbar ist, wobei der Magnet am ersten Bauteil angeordnet ist, und wobei am zweiten Bauteil eine Schneide angeordnet ist.

Das zweite Bauteil ist insbesondere relativ zum ersten Bauteil und zu einem abzutragenden Gewebe rotierbar. Anders ausgedrückt ist das medizinische Werkzeug ausgebildet, um bei einer Rotation des zweiten Bauteils relativ zum ersten Bauteil Gewebe abzutragen. Dabei rotiert das zweite Bauteil auch relativ zu einer Handhabungseinrichtung am proximalen Ende eines medizinischen Instruments, dessen dauernder oder vorübergehender Bestandteil des medizinische Werkzeug sein kann.

Das erste Bauteil ist oder umfasst insbesondere einen Außenschaft oder ein distales Ende eines Außenschafts des medizinischen Werkzeugs. Das zweite Bauteil ist oder umfasst insbesondere einen Innenschaft, der relativ zum Außenschaft rotierbar ist, und an dem eine mit dem Innenschaft rotierbare Schneide angeordnet ist. An dem Außenschaft kann ebenfalls eine Schneide angeordnet sein, die beim Abtragen des Gewebes im Wesentlichen ruht, zumindest nicht rotiert.

Die Anordnung des Magneten an einem während des Abtragens von Gewebe im Wesentlichen ruhenden Bauteil kann ein gutes Verhältnis zwischen einem Querschnitt eines Lumens in einem rotierenden Bauteil und einem Gesamtquerschnitt des medizinischen Werkzeugs ermöglichen. Ferner kann die Anordnung des Magneten an einem während des Abtragens von Gewebe im Wesentlichen ruhenden Bauteil mehr Bauraum für den Magneten und gegebenenfalls für ein Reservoir zum Aufnehmen von durch den Magneten angezogenen und gesammelten Partikeln ermöglichen. Dadurch kann die Leistungsfähigkeit hinsichtlich des Sammelns und Entfernens magnetischer oder magnetisierbarer Partikel insbesondere höher sein als bei einer Anordnung des Magneten an einem beim Abtragen von Gewebe rotierenden oder anders bewegten Bauteil.

Bei einem medizinischen Werkzeug, wie es hier beschrieben ist, ist der Magnet insbesondere angeordnet und ausgebildet, um während eines Abtragens von Gewebe Partikel zu sammeln.

Dazu ist der Magnet insbesondere so neben der Abtrageinrichtung angeordnet, dass eine mittels der Abtrageinrichtung bearbeitete (insbesondere ebene) Fläche gleichzeitig dem Magneten gegenüber und in dessen magnetischen Nahfeld liegt.

Bei einem medizinischen Werkzeug, wie es hier beschrieben ist, ist der Magnet insbesondere angeordnet und ausgebildet, um nach einem Abtragen von Gewebe in einem separaten Verfahrensschritt Partikel zu sammeln.

Dazu ist der Magnet insbesondere so angeordnet, dass zunächst mittels der Abtrageinrichtung Gewebe von einer Oberfläche abgetragen werden kann, und dass nach einer Rotation des medizinischen Werkzeugs um seine Längsachse magnetische oder magnetisierbare Partikel an der bearbeiteten Oberfläche gesammelt werden können, indem das medizinische Werkzeug ein zweites Mal über die bearbeitete Oberfläche geführt wird.

Ein medizinisches Werkzeug, wie es hier beschrieben ist, umfasst insbesondere ferner ein Reservoir zum Aufnehmen von durch den Magneten angezogenen und gesammelten Partikeln.

Das Reservoir ist insbesondere in Gestalt einer Ausnehmung bzw. Vertiefung bzw. eines zu einer Oberfläche des medizinischen Werkzeugs hin offenen Hohlraums ausgebildet. Insbesondere bildet zumindest eine Polfläche des Magneten zumindest einen Teil einer inneren Oberfläche des Reservoirs. Die Polfläche oder die Polflächen des Magneten sind in dem Reservoir insbesondere so angeordnet, dass sie von einer Ebene, die durch den Rand einer das Reservoir bildenden Ausnehmung definiert ist, beabstandet bzw. gegenüber dieser Ebene zurückgesetzt sind. Die Polfläche oder die Polflächen des Magneten können in diesem Fall Gewebe, über das das medizinische Werkzeug geführt wird, nicht oder nur im Fall hinreichender Elastizität des Gewebes berühren. Vom Magneten angezogene und gesammelte Partikel können deshalb nicht oder nicht ohne Weiteres wieder durch eine Gewebeoberfläche abgestreift werden, sondern verbleiben im Reservoir.

Bei einem medizinischen Werkzeug mit einem Reservoir, wie es hier beschrieben ist, weist das Reservoir insbesondere eine Öffnung an einer Seite des medizinischen Werkzeugs, die von einem Wirkbereich der Abtrageinrichtung des medizinischen Werkzeugs abgewandt ist, auf.

Der Wirkbereich der Abtrageinrichtung ist insbesondere die unmittelbare Umgebung einer feststehenden Schneide und/oder der Raumbereich, in dem eine rotierbare oder anderweitig bewegbare Schneide bewegbar ist. Die Anordnung der Öffnung des Reservoirs einerseits und des Wirkbereichs andererseits an voneinander abgewandten Seiten des medizinischen Werkzeugs ermöglicht eine selektive Anwendung des medizinischen Werkzeugs entweder zum Abtragen von Gewebe oder zum Anziehen und Sammeln von magnetischen oder magnetisierbaren Partikeln. Durch Rotation des medizinischen Werkzeugs um seine Längsachse kann ausgewählt werden, ob Gewebe abgetragen oder ob magnetische oder magnetisierbare Partikel angezogen und gesammelt werden sollen.

Die Öffnung des Reservoirs und der Wirkbereich der Abtrageinrichtung können an entgegengesetzten Seiten des medizinischen Werkzeugs angeordnet sein. In diesem Fall muss das medizinische Werkzeug im Wesentlichen um 180 Grad rotiert werden, um von einer der beiden Funktionen (einerseits Abtragen und andererseits Anziehen/Sammeln) zur anderen überzugehen. Alternativ ist das medizinische Werkzeug so ausgebildet, dass für einen Übergang zwischen den beiden Funktionen eine Rotation um einen kleineren Winkel, beispielsweise um 90 Grad, erforderlich ist.

Bei einem medizinischen Werkzeug, wie es hier beschrieben ist, ist insbesondere zumindest eine Polfläche des Magneten an einer von einem Wirkbereich des medizinischen Werkzeugs abgewandten Seite des medizinischen Werkzeugs angeordnet.

Die Anordnung zumindest einer Polfläche des Magneten einerseits und des Wirkbereichs andererseits an voneinander abgewandten Seiten des medizinischen Werkzeugs ermöglicht eine selektive Anwendung des medizinischen Werkzeugs entweder zum Abtragen von Gewebe oder zum Anziehen und Sammeln von magnetischen oder magnetisierbaren Partikeln. Durch Rotation des medizinischen Werkzeugs um seine Längsachse kann ausgewählt werden, ob Gewebe abgetragen oder ob magnetische oder magnetisierbare Partikel angezogen und gesammelt werden sollen.

Die Polfläche des Magneten und der Wirkbereich der Abtrageinrichtung können an entgegengesetzten Seiten des medizinischen Werkzeugs angeordnet sein. In diesem Fall muss das medizinische Werkzeug im Wesentlichen um 180 Grad rotiert werden, um von einer der beiden Funktionen (einerseits Abtragen und andererseits Anziehen/Sammeln) zur anderen überzugehen. Alternativ ist das medizinische Werkzeug so ausgebildet, dass für einen Übergang zwischen den beiden Funktionen eine Rotation um einen kleineren Winkel, beispielsweise um 90 Grad, erforderlich ist.

Bei einem medizinischen Werkzeug, wie es hier beschrieben ist, weist die Abtrageinrichtung insbesondere ein magnetisches oder magnetisierbares Material auf.

Wie bereits erwähnt, ist mit einem magnetischen oder magnetisierbaren Material ein nicht lediglich diamagnetisches Material gemeint. Insbesondere ist ein magnetisches oder magnetisierbares Material ein paramagnetisches, ferromagnetisches, antiferromagnetisches oder ferrimagnetisches Material. Die Verwendung eines magnetischen oder magnetisierbaren Materials an der Abtrageinrichtung hat zur Folge, dass von der Abtrageinrichtung gelöste Partikel aufgrund ihrer magnetischen oder magnetisierbaren Eigenschaft vom Magneten angezogen und gesammelt werden können. Somit müssen keine oder wesentlich weniger aus Abrieb resultierende Partikel als bisher in einem Operationsgebiet zurückbleiben.

Bei einem medizinischen Werkzeug, wie es hier beschrieben ist, weist die Abtrageinrichtung insbesondere eine geometrisch bestimmte Schneide auf.

Die Abtrageinrichtung umfasst insbesondere einen Fräser mit einer, zwei, drei, vier oder mehr geometrisch bestimmten Schneiden. Alternativ oder zusätzlich kann die Abtrageinrichtung eine Schleifeinrichtung mit geometrisch unbestimmten Schneiden aufweisen. Bei einem medizinischen Werkzeug, wie es hier beschrieben ist, weist die Abtrageinrichtung insbesondere zwei Schneiden auf, die ausgebildet und angeordnet sind, um zum Abtragen von Gewebe aneinander vorbei bewegt zu werden.

Insbesondere sind eine oder mehrere Schneiden an einem feststehenden Außenschaft und eine oder mehrere Schneiden an einem Innenschaft angeordnet. Der Innenschaft ist insbesondere im Außenschaft angeordnet und ausgebildet, um um eine gemeinsame Längsachse des Außenschafts und des Innenschafts zu rotieren. Zwischen den zwei oder mehr aneinander vorbei bewegten Schneiden wird das Gewebe durch Scheren bzw. Scherschneiden durchtrennt.

Ein medizinisches Instrument umfasst ein medizinisches Werkzeug, wie es hier beschrieben ist.

Bei einem beispielweisen Verfahren zum Abtragen von Gewebe werden Gewebe mittels eines medizinischen Werkzeugs abgetragen und beim Abtragen entstehende magnetische oder magnetisierbare Partikel mittels eines Magneten an dem medizinischen Werkzeug angezogen.

Das beispielweise Verfahren wird insbesondere mit einem medizinischen Werkzeug oder einem medizinischen Instrument, wie es hier beschrieben ist, ausgeführt.

Bei einem beispielweisen Verfahren werden die Partikel insbesondere während oder nach dem Abtragen angezogen.

Bei einem beispielweisen Verfahren werden die Partikel insbesondere in einem Reservoir gesammelt.

Ein beispielweises Verfahren umfasst ferner zwischen dem Schritt des Abtragens und dem Schritt des Anziehens einen Schritt des Rotierens des medizinischen Werkzeugs.

Zwischen dem Abtragen und dem Anziehen wird nicht lediglich ein Teil des medizinischen Werkzeugs rotiert, beispielsweise ein rotierbarer Innenschaft oder ein rotierbarer Fräser. Vielmehr wird das gesamte medizinische Werkzeug rotiert. Insbesondere umfasst das Rotieren des medizinischen Werkzeugs ein Rotieren eines Außenschafts des medizinischen Werkzeugs.

Beim Rotieren des medizinischen Werkzeugs nach dem Abtragen von Gewebe und vor dem Anziehen von Partikeln wird insbesondere ein Wirkbereich des medizinischen Werkzeugs von einer Oberfläche, von der Gewebe abgetragen wurde, weg gedreht. Gleichzeitig wird der Magnet zu der Oberfläche, von der Gewebe abgetragen wurde, hin gedreht.

### Kurzbeschreibung der Figuren

Nachfolgend werden Ausführungsformen anhand der beigefügten Figuren näher erläutert. Es zeigen:
Figur 1 eine schematische Darstellung eines medizinischen Instruments;
Figur 2 eine schematische Darstellung eines medizinischen Werkzeugs;
Figur 3 eine schematische Schnittdarstellung des medizinischen Werkzeugs aus Figur 2;
Figur 4 eine weitere schematische Schnittdarstellung des medizinischen Werkzeugs aus den Figuren 2 und 3;
Figur 5 eine schematische Schnittdarstellung eines weiteren medizinischen Werkzeugs;
Figur 6 eine schematische Schnittdarstellung eines weiteren medizinischen Werkzeugs;
Figur 7 eine schematische Schnittdarstellung eines weiteren medizinischen Werkezugs;
Figur 8 eine schematische Schnittdarstellung eines weiteren medizinischen Werkzeugs;
Figur 9 ein schematisches Flussdiagramm eines beispielweisen Verfahrens zum Abtragen von Gewebe.

### Beschreibung der Ausführungsformen

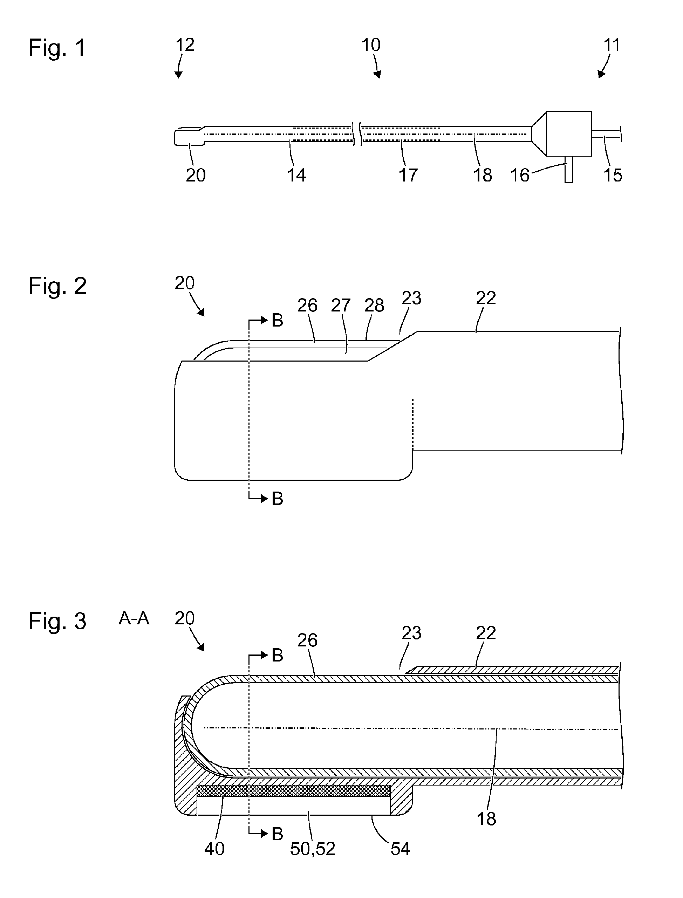

Figur 1 zeigt eine schematische Darstellung eines medizinischen Instruments 10 mit einem proximalen Ende 11, einem distalen Ende 12 und einem Schaft 14, der sich im Wesentlichen vom proximalen Ende 11 bis zum distalen Ende 12 erstreckt. Der Schaft 14 ist insbesondere gerade und starr und weist eine zylindrische, insbesondere kreiszylindrische Mantelfläche auf. Alternativ kann der Schaft zumindest abschnittsweise gekrümmt und/oder zumindest abschnittsweise flexibel sein und/oder einen variierenden Querschnitt aufweisen.

Am proximalen Ende 11 des medizinischen Instruments 10 ist eine Antriebseinrichtung 15 vorgesehen. Die Antriebseinrichtung 15 ist insbesondere eine flexible Welle oder eine Kupplung zum Koppeln des medizinischen Instruments mit einer flexiblen Welle. Mittels der flexiblen Welle ist das proximale Ende 11 des medizinischen Instruments 10 mit einem Motor, insbesondere einem Elektromotor verbunden oder verbindbar. Alternativ kann das medizinische Instrument 10 selbst am proximalen Ende 11 einen Motor aufweisen, insbesondere einen Elektromotor, einen pneumatischen Motor oder einen hydraulischen Motor.

Ferner weist das medizinische Instrument 10 am proximalen Ende 11 einen Absaugstutzen 16 bzw. eine Kupplung zur lösbaren Verbindung des medizinischen Instruments 10 mit einer Pumpe oder Absaugeinrichtung bzw. einer Quelle von Unterdruck zum Absaugen von fließfähigem Material auf.

Im Schaft 14 weist das medizinische Instrument eine Welle 17 auf, von der in Figur 1 nur ein Abschnitt durch gestrichelte Linien angedeutet ist. Die Welle 17 ist im Schaft 14 und insbesondere um eine gemeinsame Symmetrieachse 18 des Schafts 14 und der Welle 17 rotierbar. Die Welle 17 ist am proximalen Ende 11 des medizinischen Instruments 10 unmittelbar oder mittelbar mit der Antriebseinrichtung 15 gekoppelt, so dass die Antriebseinrichtung 15 die Welle 17 um ihre Symmetrieachse 18 rotieren kann. Wenn die Welle 17 hohl bzw. rohrförmig ist, ist ihr Lumen am distalen Ende 11 des medizinischen Instruments 10 fluidisch mit dem Absaugstutzen 16 gekoppelt, so dass Fluide im Inneren der Welle 17 vom distalen Ende 12 zum proximalen Ende 11 und zum Absaugstutzen 16 gesaugt werden können.

Am distalen Ende 12 weist das medizinische Instrument 10 ein medizinisches Werkzeug 20 zum Abtragen von Gewebe auf. Ausführungsbeispiele des Werkzeugs 20 sind unten mit Bezug auf die Figuren 2 bis 8 dargestellt.

Figur 2 zeigt eine schematische Darstellung einer Ausführungsform des Werkzeugs 20 am distalen Ende 12 des medizinischen Instruments aus Figur 1. Das medizinische Werkzeug 20 umfasst einen Außenschaft 22, der einstückig mit dem Schaft 14 des medizinischen Instruments 10 (vergleiche Figur 1) ausgebildet sein kann. Insbesondere bildet das distale Ende des Schafts 14 des medizinischen Instruments 10 den Außenschaft 22 des medizinischen Werkzeugs 20. Alternativ ist der Außenschaft 22 des medizinischen Werkzeugs 20 mit dem Schaft 14 des medizinischen Instruments 10 zerstörungsfrei lösbar mechanisch gekoppelt, beispielsweise über Kupplungseinrichtungen.

Der Außenschaft 22 weist eine fensterförmige Öffnung 23 auf, durch die hindurch ein Teil eines rotierbaren Innenschafts 26 sichtbar ist. Aufgrund der in Figur 2 dargestellten Winkelposition des Innenschafts 26 ist eine Schneide 28 am Rand einer Öffnung 27 am Innenschaft 26 sichtbar. Die Schneide 28 verläuft parallel oder im Wesentlichen parallel zur Zeichenebene der Figur 2.

Figur 3 zeigt eine schematische Schnittdarstellung des medizinischen Werkzeugs 20 aus Figur 2. Die dargestellte Schnittebene A-A ist parallel zu den Zeichenebenen der Figuren 1 und 2 und enthält die Längsachse 18 des medizinischen Instruments 10 bzw. des Schafts 14 des medizinischen Instruments 10 (vgl. Figur 1). Der Innenschaft 26 ist im Wesentlichen kreiszylindrisch und im Außenschaft 22 spiel- und reibungsarm um die Längsachse 18 rotierbar.

Ein Magnet 40 ist in einem Reservoir 50 bzw. in einem Innenraum 52 des Reservoirs 50 angeordnet. Das Reservoir 50 weist eine Öffnung 54 an einer von der Öffnung 23 am Außenschaft 22 abgewandten Seite auf. Anders ausgedrückt liegen die Öffnung 23 im Außenschaft 22 und die Öffnung 54 des Reservoirs 50 an aneinander entgegengesetzten Seiten des medizinischen Werkzeugs 20, insbesondere des Außenschafts 22.

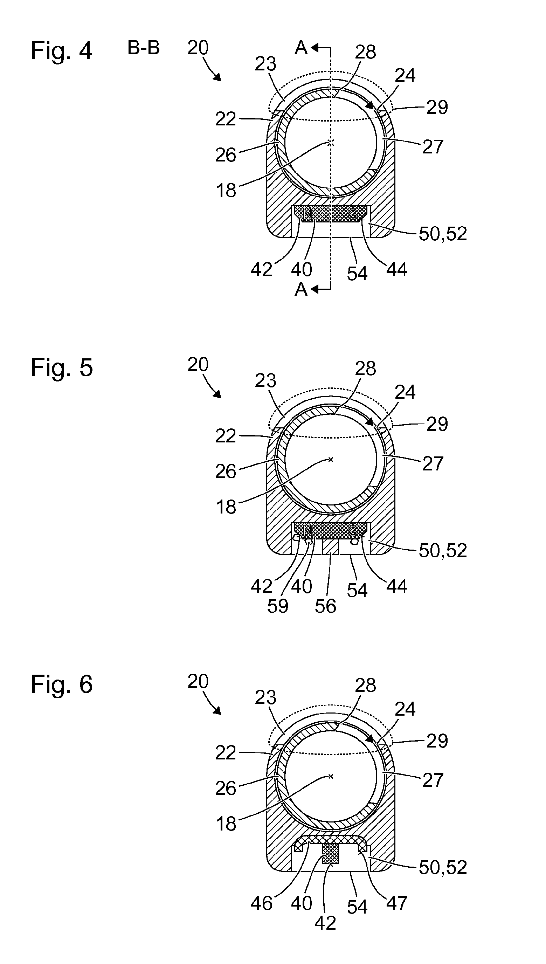

Figur 4 zeigt eine weitere schematische Schnittdarstellung des medizinischen Werkzeugs 20 aus den Figuren 2 und 3. Die Schnittebene B-B der Figur 4 ist senkrecht zur Zeichenebene der Figur 2, senkrecht zur Schnittebene A-A der Figur 3 und senkrecht zur Längsachse 18. Die Lage der Schnittebene B-B der Figur 4 ist in den Figuren 2 und 3 angedeutet. Die Lage der Schnittebene A-A der Figur 3 ist in Figur 4 angedeutet.

In Figur 4 sind einige bereits oben anhand der Figuren 2 und 3 dargestellte Merkmale des medizinischen Werkzeugs 20 erkennbar. Insbesondere sind am Rand der Öffnung 23 am Außenschaft 22 eine Schneide 24 und am Rand der Öffnung 27 am Innenschaft 26 eine Schneide 28 erkennbar. Bei dem dargestellten Beispiel sind die jeweils gegenüberliegenden Ränder der Öffnung 23 am Außenschaft 22 und der Öffnung 27 am rotierbaren Innenschaft 26 ebenfalls als Schneiden ausgebildet. Durch einen Pfeil ist in Figur 4 eine Rotationsbewegung des Innenschafts 26 angedeutet, bei der durch die Öffnung 23 am Außenschaft 22 und die Öffnung 27 am Innenschaft 26 in den Innenraum des Innenschafts 26 gesaugtes Gewebe durch eine Scherbewegung der beiden Schneiden 24, 28 relativ zueinander durchtrennt wird.

Ferner ist in Figur 4 die Anordnung des Reservoirs 50 und der Öffnung 54 des Reservoirs 50 an einer von der Öffnung 23 am Außenschaft 22 abgewandten Seite erkennbar. Die Öffnung 23 am Außenschaft 22 und die Öffnung 54 am Reservoir 50 sind somit, wie bereits oben im Zusammenhang mit Figur 3 erwähnt, in entgegengesetzte Richtungen orientiert.

Im Innenraum 52 des Reservoirs 50 ist der Magnet 40 symmetrisch zur Schnittebene A-A der Figur 3 angeordnet. Nordpol und Südpol des Magneten 40 und zugeordnete Polflächen 42, 44 sind entsprechend symmetrisch zur Schnittebene A-A angeordnet. Der Magnet 40 ist im Wesentlichen quaderförmig. Die Polflächen 42, 44 des Magneten 40 umfassen Abschrägungen bzw. Fasen, die teilweise der Öffnung 54 des Reservoirs 50 zugewandt sind. Der Magnet 40 ist insbesondere so magnetisiert, dass das an den Polflächen 42, 44 aus- bzw. eintretende Magnetfeld in möglichst hohem Maße im Innenraum 52 des Reservoirs 50 und in dem über die Öffnung 54 des Reservoirs 50 angrenzenden Raum und in möglichst geringem Maße im Außenschaft 22 konzentriert ist. Dazu kann der Magnet 40 eine von den Darstellungen in den Figuren 3 und 4 abweichende Gestalt aufweisen.

Der Magnet 40 füllt die Ausnehmung im Außenschaft, die das Reservoir 50 bildet, nur teilweise aus. Der nicht vom Magneten 40 ausgefüllte Raumbereich der Ausnehmung wird als Innenraum 52 des Reservoirs 50 bezeichnet. Die Polflächen 42, 44 des Magneten 40 bilden einen Teil der inneren Oberfläche des Reservoirs 50.

Der Magnet 40 ist gegenüber der Öffnung 54 des Reservoirs 50 zurückgesetzt angeordnet. Anders ausgedrückt ist der Magnet 40 von der Öffnung 54 des Reservoirs 50 beabstandet. Insbesondere liegt der Rand der Öffnung 54 des Reservoirs 50 in einer Ebene, die von dem Magneten 40 beabstandet ist. An den Polflächen 42, 44 des Magneten 40 haftende Partikel berühren deshalb nicht oder nicht ohne Weiteres Gewebe, über das das Reservoir 50 mit dem Magneten 40 geführt wird. Dadurch ist das Risiko vermindert, dass an den Polflächen 42, 44 haftende Partikel durch Gewebe von den Polflächen 42, 44 abgestreift werden.

Das in den Figuren 2 bis 4 dargestellte medizinische Werkzeug kann abwechselnd verwendet werden, um mittels der Schneiden 24, 28 Gewebe von einer Oberfläche abzutragen. Dazu wird die zu bearbeitende Oberfläche bzw. die Oberfläche, von der Gewebe abzutragen ist, in den in Figur 4 durch eine gestrichelte Kontur 29 angedeuteten Wirkbereich der Schneiden 24, 28 gebracht. Hinsichtlich der Wechselwirkung mit Gewebe können die Schneiden 24, 28 bzw. die durch die Schneiden 24, 28 gebildete Abtrageinrichtung (auch abweichend von der Darstellung in den Figuren 2 bis 4) Eigenschaften und Merkmale aufweisen, die denen der eingangs erwähnten DE 10 2006 034 756 A1 und der DE 10 2009 010 561 A1 ähneln.

Nach einer Rotation des medizinischen Werkzeugs 20 um 180 Grad um die Längsachse 18 kann die Öffnung 54 des Reservoirs 50 über die zuvor bearbeitete Oberfläche geführt werden. Der Magnet 40 zieht magnetische oder magnetisierbare Partikel, die beispielsweise durch Verschleiß am Außenschaft 22 und am Innenschaft 26 erzeugt wurden, an und sammelt diese im Reservoir 50.

Figur 5 zeigt eine schematische Schnittdarstellung eines weiteren medizinischen Werkzeugs 20, das in einigen Merkmalen und Eigenschaften dem oben anhand der Figuren 2 bis 4 dargestellten medizinischen Werkzeug ähnelt. Nachfolgend sind nur Merkmale und Eigenschaften dargestellt, in denen das medizinische Werkzeug 20 aus Figur 5 sich von dem oben anhand der Figuren 2 bis 4 dargestellten medizinischen Werkzeug unterscheidet. Die Schnittebene der Figur 5 entspricht der Schnittebene der Figur 4.

Im Innenraum 52 des Reservoirs 50 ist ein Steg 56 angeordnet, der vom Magneten 40 bis zu der durch den Rand der Öffnung 54 des Reservoirs 50 definierten Ebene reicht. Der Steg 56 weist insbesondere ein nicht-magnetisches und nicht-magnetisierbares Material auf. Der Steg 56 unterteilt das Reservoir 50 in zwei im Wesentlichen symmetrische Teilbereiche und reduziert das Risiko, dass elastisches Gewebe weit in den Innenraum 52 des Reservoirs 50 eindringt und dort die Polflächen 42, 44 des Magneten 40 berührt. Dadurch reduziert der Steg 56 das Risiko, dass an den Polflächen 42, 44 haftende Partikel 59 durch Gewebe wieder von den Polflächen 42, 44 abgestreift werden.

Ferner reduziert der Steg 56 die Wahrscheinlichkeit, dass magentische oder magnetisierbare Partikel eine Brücke zwischen den Polflächen 42, 44 des Magneten 40 bilden und diese magnetisch kurzschließen. Der Steg 56 trägt so zur längeren Aufrechterhaltung der durch den Magneten auf magnetische oder magnetisierbare Partikel 59 ausgeübten Anziehungskraft bei.

Figur 6 zeigt eine schematische Schnittdarstellung eines weiteren medizinischen Werkzeugs 20, das in einigen Merkmalen und Eigenschaften den oben anhand der Figuren 2 bis 5 dargestellten medizinischen Werkzeugen ähnelt. Nachfolgend sind lediglich Merkmale und Eigenschaften dargestellt, in denen das medizinische Werkzeug aus Figur 6 sich von den oben anhand der Figuren 2 bis 5 dargestellten medizinischen Werkzeugen unterscheidet. Die Schnittebene der Figur 6 entspricht den Schnittebenen der Figuren 4 und 5.

Im Reservoir 50 ist angrenzend an einen Pol des Magneten 40 ein Magnetflussleiter 46 aus einem magnetisierbaren Material angeordnet. Der Magnet 40 und der Magnetflussleiter 46 sind in einer im dargestellten Querschnitt T-förmigen und symmetrischen Konfiguration angeordnet. Neben der beispielsweise den Nordpol des Magneten 40 bildenden Polfläche 42 am Magneten 40 selbst, bilden deshalb zwei weitere Polflächen 47 (im genannten Beispiel: Südpole) Teile der inneren Oberfläche des Innenraums 52 des Reservoirs 50. Die im Vergleich zu den medizinischen Werkzeugen aus den Figuren 2 bis 5 erhöhte Anzahl von Polflächen 42, 47 bei dem medizinischen Werkzeug 20 aus Figur 6 kann das Sammeln magnetischer oder magnetisierbarer Partikel vereinfachen.

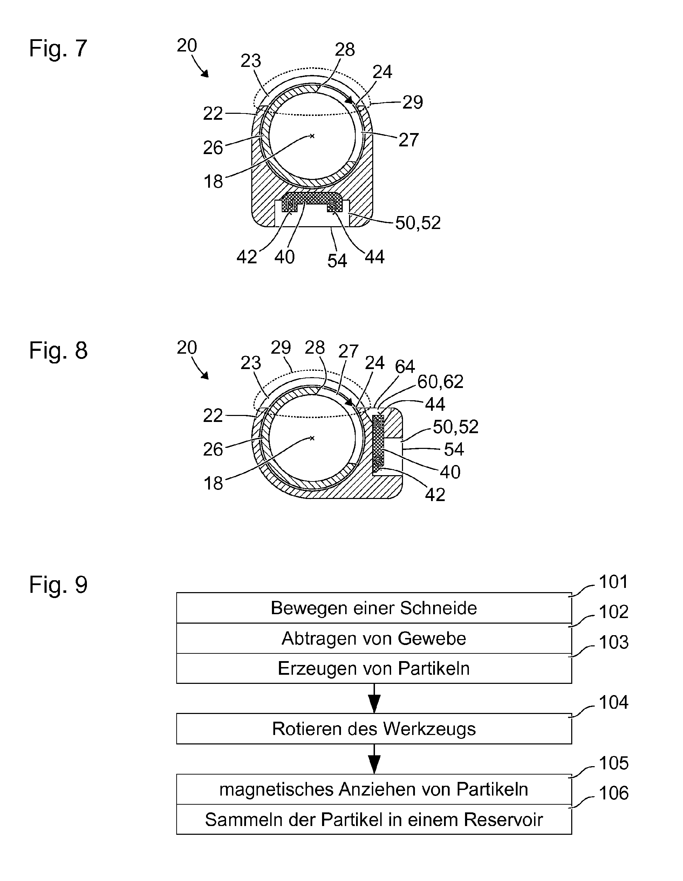

Figur 7 zeigt eine schematische Schnittdarstellung eines weiteren medizinischen Werkzeugs 20, das in einigen Merkmalen und Eigenschaften den oben anhand der Figuren 2 bis 6 dargestellten medizinischen Werkzeugen ähnelt. Nachfolgend sind lediglich Merkmale und Eigenschaften dargestellt, in denen das medizinische Werkzeug aus Figur 7 sich von den oben anhand der Figuren 2 bis 6 dargestellten medizinischen Werkzeugen unterscheidet. Die Schnittebene der Figur 7 entspricht den Schnittebenen der Figuren 4 bis 6.

Bei dem medizinischen Werkzeug 20 aus Figur 7 weist der Magnet 40 einen im Wesentlichen U-förmigen Querschnitt auf. Die Polflächen 42, 44 des Magneten 40 sind im Wesentlichen parallel zueinander und parallel zu der durch den Rand der Öffnung 54 des Reservoirs 50 definierten Ebene. Diese Gestalt des Magneten 40 kann dazu beitragen, dass das vom Magneten 40 erzeugte Magnetfeld im Wesentlichen innerhalb des Innenraums 52 des Reservoirs 50 und in den über die Öffnung 54 angrenzenden Raumbereich konzentriert ist. Dadurch können die dargestellten Sammeleigenschaften des Magneten 40 verbessert werden.

Figur 8 zeigt eine schematische Schnittdarstellung eines weiteren medizinischen Werkzeugs 20, das in einigen Merkmalen und Eigenschaften den oben anhand der Figuren 2 bis 7 dargestellten medizinischen Werkzeugen ähnelt. Nachfolgend sind lediglich Merkmale und Eigenschaften dargestellt, in denen das medizinische Werkzeug aus Figur 8 sich von den oben anhand der Figuren 2 bis 7 dargestellten medizinischen Werkzeugen unterscheidet. Die Schnittebene der Figur 8 entspricht den Schnittebenen der Figuren 4 bis 7.

Das medizinische Werkzeug 20 aus Figur 8 weist zwei voneinander räumlich getrennte und beabstandete Reservoire 50, 60 auf. Der Magnet 40 ist so angeordnet, dass ein Pol des Magneten 40 am ersten Reservoir 50 und der andere Pol des Magneten 40 am zweiten Reservoir 60 liegt. Eine erste Polfläche 42 des Magneten 40 bildet einen Teil der inneren Oberfläche des Innenraums 52 des ersten Reservoirs 50. Eine zweite Polfläche 44 des Magneten 40 bildet einen Teil der inneren Oberfläche des Innenraums 62 des zweiten Reservoirs 60. Beide Polflächen 42, 44 sind gegenüber den Öffnungen 54, 64 der zugeordneten Reservoire 50, 60 zurückgesetzt im oben beschriebenen Sinne.

Die Reservoire 50, 60 und ihre Öffnungen 54, 64 sind in unterschiedliche Richtungen orientiert. Die Öffnung 54 des ersten Reservoirs 50 ist so orientiert, dass nach einem Abtrag von Gewebe mittels der durch die Schneiden 24, 28 gebildeten Abtrageinrichtung das medizinische Werkzeug 20 um einen Winkel von 90 Grad um die Längsachse 18 rotiert werden muss, um mittels der ersten Polfläche 42 des Magneten 40 magnetische oder magnetisierbare Partikel an der bearbeiteten Oberfläche im ersten Reservoir 50 zu sammeln. Das zweite Reservoir 60 und die Öffnung 62 des zweiten Reservoirs 60 sind so angeordnet und orientiert, dass bereits beim Abtragen von Gewebe von einer Oberfläche mittels der durch die Schneiden 24, 28 gebildeten Abtrageinrichtung magnetische oder magnetisierbare Partikel von der Oberfläche im zweiten Reservoir 60 gesammelt werden können. Anders ausgedrückt liegen das zweite Reservoir 60 und die Öffnung 64 des zweiten Reservoirs 60 neben der Abtrageinrichtung 24, 28 bzw. neben deren Wirkbereich 29.

Abweichend von der Darstellung in Figur 8 können für jedes Reservoir 50, 60 ein oder mehrere eigene Magneten vorgesehen sein. Dabei bilden insbesondere alle Polflächen eines ersten Magneten innere Oberflächen des Innenraums 52 des ersten Reservois 50 und alle Polflächen eines zweiten Magneten innere Oberflächen des Innenraums 62 des zweiten Reservois 60. Ferner kann symmetrisch zum zweiten Reservoir 60 ein drittes Reservoir vorgesehen sein, so dass zu beiden Seiten der Abtrageinrichtung 24, 28 neben dieser jeweils ein Reservoir angeordnet ist. Mit beiderseits neben der Abtrageinrichtung 24, 28 angeordneten Reservoirs können beim Abtragen von Gewebe entstehende magnetische oder magnetisierbare Partikel unabhängig davon gesammelt werden, in welche Richtung das Werkzeug 20 bewegt wird (in Figur 8: nach links oder nach rechts).

Merkmale der anhand der Figuren 2 bis 8 dargestellten medizinischen Werkzeuge sind teilweise anders kombinierbar als anhand der Figuren 2 bis 8 dargestellt. Ferner können die Abmessungen und Querschnitte des Magneten 40, der Reservoire 50, 60, des Außenschafts 22, des rotierbaren Innenschafts 26 und der Schneiden 24, 28 von den Darstellungen in den Figuren 2 bis 8 abweichen. Anstelle eines Magneten können zwei oder mehr Magneten an einem oder mehreren Reservoire 50, 60 vorgesehen sein. Der rotierbare Innenschaft 26 kann anstelle einer Öffnung 27 mehrere Öffnungen mit einer entsprechenden Mehrzahl von Schneiden 28 aufweisen.

Figur 9 zeigt ein schematisches Flussdiagramm eines beispielweisen Verfahrens zum Abtragen von Gewebe. Das beispielweise Verfahren ist mittels eines medizinischen Werkzeugs bzw. eines medizinischen Instruments ausführbar, das von den Darstellungen anhand der Figuren 1 bis 8 abweichende Merkmale und Eigenschaften aufweist. Trotzdem werden nachfolgend zur Verbesserung des Verständnisses beispielhaft Bezugszeichen aus den Figuren 1 bis 8 verwendet.

Bei einem ersten Schritt 101 wird eine Schneide 28 bewegt, insbesondere rotiert. Bei einem zweiten Schritt 102 wird Gewebe mittels der bewegten Schneide 28 abgetragen. Am zweiten Schritt 102 kann ferner eine feststehende Schneide 24 beteiligt sein, wobei insbesondere Gewebe durch eine Scherbewegung der Schneiden 24, 28 durchtrennt wird. Bei einem dritten Schritt 103 werden magnetische oder magnetisierbare Partikel 59 erzeugt, insbesondere durch Verschleiß bzw. Abrieb an der Schneide 28 oder an einer anderen Stelle eines medizinischen Werkzeugs 20, dessen Bestandteil die Schneide 28 ist. Der erste Schritt 101, der zweite Schritt 102 und der dritte Schritt 103 werden insbesondere gleichzeitig ausgeführt, wobei aufgrund der Rotation der Schneide 28 Gewebe abgetragen wird und dabei Abrieb in Form von Partikeln entsteht.

Bei einem optionalen vierten Schritt 104 wird das Werkzeug 20 rotiert, insbesondere um seine Längsachse und insbesondere um einen Winkel von 180 Grad.

Bei einem fünften Schritt 105 werden beim dritten Schritt 103 erzeugte magnetische oder magnetisierbare Partikel 59 mittels eines Magneten 40 angezogen. Bei einem sechsten Schritt 106 werden die beim fünften Schritt 105 angezogenen Partikel 59 in einem Reservoir 50, 60 gesammelt. Der fünfte Schritt 105 und der sechste Schritt 106 werden mit dem gleichen medizinischen Werkzeug 20 ausgeführt, mit dem der erste Schritt 101, der zweite Schritt 102 und der dritte Schritt 103 ausgeführt werden. Der fünfte Schritt 105 und der sechste Schritt 106 werden insbesondere gleichzeitig oder im Wesentlichen gleichzeitig ausgeführt, wobei die magnetischen oder magnetisierbaren Partikel 59 durch den Magneten 40 in das Reservoir 50, 60 gezogen werden.

Der erste Schritt 101, der zweite Schritt 102 und der dritte Schritt 103 einerseits sowie der fünfte Schritt 105 und der sechste Schritt 106 andererseits können gleichzeitig oder unmittelbar nacheinander in einer kontinuierlichen Anwendung des medizinischen Werkzeugs 20 ausgeführt werden. In diesem Fall entfällt der vierte Schritt 104. Alternativ werden der fünfte Schritt 105 und der sechste Schritt 106 separat ausgeführt, nachdem das medizinische Werkzeug 20 beim vierten Schritt 104 gegenüber der Orientierung beim ersten Schritt 101, beim zweiten Schritt 102 und beim dritten Schritt 103 um einen vorbestimmten Winkel (beispielsweise 180 Grad im Falle der oben anhand der Figuren 2 bis 8 dargestellten Beispielen oder 90 Grad im Falle des oben anhand der Figur 8 dargestellten Beispiels) rotiert wurde.

### Bezugszeichen

- 10: Medizinisches Instrument
- 11: proximales Ende des medizinischen Instruments 10
- 12: distales Ende des medizinischen Instruments 10
- 14: Schaft des medizinischen Instruments 10
- 15: Antriebseinrichtung am proximalen Ende 11 des medizinischen Instruments 10
- 16: Absaugstutzen
- 17: Welle zwischen Antriebseinrichtung 15 und Werkzeug 20
- 18: Symmetrieachse des Schafts 14

- 20: medizinisches Werkzeug
- 22: Außenschaft des Werkzeugs 20
- 23: Öffnung am Außenschafts des Werkzeugs 20
- 24: Schneide am Außenschaft 22
- 26: rotierbarer Innenschaft des Werkzeugs 20
- 27: Öffnung am Innenschaft 26
- 28: Schneide am rotierbaren Innenschaft 26
- 29: Wirkbereich der Schneiden 24, 28

- 40: Magnet
- 42: erste Polfläche des Magneten 40
- 44: zweite Polfläche des Magneten 40
- 46: Magnetflussleiter
- 47: Polfläche am Magnetflussleiter

- 50: Reservoir am medizinischen Werkzeug 20
- 52: Innenraum des Reservoirs 50
- 54: Öffnung des Reservoirs 50
- 56: Steg
- 59: magnetisches oder magnetisierbares Partikel

- 60: weiteres Reservoir am medizinischen Werkzeug 20
- 62: Innenraum des Reservoirs 60
- 64: Öffnung des Reservoirs 60

- 101: erster Schritt (Bewegen, insb. Rotieren einer Schneide)
- 102: zweiter Schritt (Abtragen von Gewebe)
- 103: dritter Schritt (Erzeugen von magnetischen oder magnetisierbaren Partikeln)
- 104: vierter Schritt (Rotieren des Werkzeugs)
- 105: fünfter Schritt (Anziehen der Partikel durch Magneten)
- 106: sechster Schritt (Sammeln der Partikel in Reservoir)

## Patentansprüche

1. Medizinisches Werkzeug (20) zum Abtragen von Gewebe, mit:
einer Abtrageinrichtung (24, 26, 28) zum spanenden Abtragen von Gewebe;
**dadurch gekennzeichnet, dass** das medizinische Werkzeug (20) einen Magnet (40) zum magnetischen Anziehen und Sammeln von magnetischen oder magnetisierbaren Partikeln (59) aufweist.

2. Medizinisches Werkzeug (20) nach dem vorangehenden Anspruch, bei dem der Magnet (40) an einem während eines Abtragens von Gewebe im Wesentlichen ruhenden Bauteil (22) des Werkzeugs (20) angeordnet ist.

3. Medizinisches Werkzeug (20) nach einem der vorangehenden Ansprüche, bei dem die Abtrageinrichtung ein erstes Bauteil (22) und ein zweites Bauteil (26) umfasst, zum Abtragen von Gewebe das zweite Bauteil (26) relativ zu dem ersten Bauteil (22) bewegbar ist,
der Magnet (40) am ersten Bauteil (22) angeordnet ist,
am zweiten Bauteil (26) eine Schneide (28) angeordnet ist.

4. Medizinisches Werkzeug (20) nach einem der vorangehenden Ansprüche, wobei der Magnet (40) angeordnet und ausgebildet ist, um während eines Abtragens von Gewebe Partikel (59) zu sammeln.

5. Medizinisches Werkzeug (20) nach einem der vorangehenden Ansprüche, wobei der Magnet (40) angeordnet und ausgebildet ist, um nach einem Abtragen von Gewebe in einem separaten Verfahrensschritt Partikel (59) zu sammeln.

6. Medizinisches Werkzeug (20) nach einem der vorangehenden Ansprüche, ferner mit einem Reservoir (50) zum Aufnehmen von durch den Magneten (40) angezogenen und gesammelten Partikeln (59).

7. Medizinisches Werkzeug (20) nach dem vorangehenden Anspruch, wobei das Reservoir (50) eine Öffnung (54) an einer Seite des medizinischen Werkzeugs (20), die von einem Wirkbereich (29) der Abtrageinrichtung (24, 26, 28) des medizinischen Werkzeugs (20) abgewandt ist, aufweist.

8. Medizinisches Werkzeug (20) nach einem der vorangehenden Ansprüche, wobei zumindest eine Polfläche (42, 44) des Magneten (40) an einer von einem Wirkbereich (29) des medizinischen Werkzeugs (20) abgewandten Seite des medizinischen Werkzeugs (20) angeordnet ist.

9. Medizinisches Werkzeug (20) nach einem der vorangehenden Ansprüche, bei dem die Abtrageinrichtung (24, 26, 28) ein magnetisches oder magnetisierbares Material aufweist.

10. Medizinisches Werkzeug (20) nach einem der vorangehenden Ansprüche, wobei die Abtrageinrichtung (20) zwei Schneiden (24, 28) aufweist, die ausgebildet und angeordnet sind, um zum Abtragen von Gewebe aneinander vorbei bewegt zu werden.

11. Medizinisches Instrument (10) mit einem medizinischen Werkzeug (20) nach einem der vorangehenden Ansprüche.

## Claims

1. Medical tool (20) for removing tissue, with:
a removing device (24, 26, 28) for the mechanical removal of tissue;
**characterized in that** the medical tool (20) has a magnet (40) for the magnetic attraction and collection of magnetic or magnetizable particles (59).

2. Medical tool (20) according to the preceding claim, in which the magnet (40) is arranged on a component (22) of the tool (20) that is substantially stationary during removal of tissue.

3. Medical tool (20) according to one of the preceding claims, in which
the removing device comprises a first component (22) and a second component (26),
the second component (26) is movable relative to the first component (22) in order to remove tissue,
the magnet (40) is arranged on the first component (22),
a blade (28) is arranged on the second component (26).

4. Medical tool (20) according to one of the preceding claims, wherein the magnet (40) is arranged and designed to collect particles (59) during removal of tissue.

5. Medical tool (20) according to one of the preceding claims, wherein the magnet (40) is arranged and designed to collect particles (59) in a separate method step after removal of tissue.

6. Medical tool (20) according to one of the preceding claims, also with a reservoir (50) for receiving particles (59) that have been attracted and collected by the magnet (40).

7. Medical tool (20) according to the preceding claim, wherein the reservoir (50) has an opening (54) on a side of the medical tool (20) directed away from an area of action (29) of the removing device (24, 26, 28) of the medical tool (20).

8. Medical tool (20) according to one of the preceding claims, wherein at least one pole surface (42, 44) of the magnet (40) is arranged on a side of the medical tool (20) directed away from an area of action (29) of the medical tool (20).

9. Medical tool (20) according to one of the preceding claims, in which the removing device (24, 26, 28) has a magnetic or magnetizable material.

10. Medical tool (20) according to one of the preceding claims, wherein the removing device (20) has two blades (24, 28) which are designed and arranged to be moved past each other in order to remove tissue.

11. Medical instrument (10) with a medical tool (20) according to one of the preceding claims.

## Revendications

1. Outil médical (20) pour l'enlèvement de tissus, comprenant :
un dispositif d'enlèvement (24, 26, 28) pour l'enlèvement de tissus par enlèvement de matière ;
**caractérisé en ce que** l'outil médical (20) présente un aimant (40) pour attirer et rassembler magnétiquement des particules magnétiques ou magnétisables (59).

2. Outil médical (20) selon la revendication précédente, dans lequel l'aimant (40) est disposé sur un composant (22) de l'outil (20) essentiellement au repos pendant un enlèvement de tissus.

3. Outil médical (20) selon l'une quelconque des revendications précédentes, dans lequel le dispositif d'enlèvement comprend un premier composant (22) et un deuxième composant (26), le deuxième composant (26), pour l'enlèvement de tissus, pouvant être déplacé par rapport au premier composant (22),
l'aimant (40) étant disposé sur le premier composant (22),
une arête de coupe (28) étant disposée sur le deuxième composant (26).

4. Outil médical (20) selon l'une quelconque des revendications précédentes, dans lequel l'aimant (40) est disposé et réalisé pour rassembler des particules (59) pendant un enlèvement de tissus.

5. Outil médical (20) selon l'une quelconque des revendications précédentes, dans lequel l'aimant (40) est disposé et réalisé pour rassembler des particules (59) après un enlèvement de tissus dans une étape de procédé séparée.

6. Outil médical (20) selon l'une quelconque des revendications précédentes, comprenant en outre un réservoir (50) pour recevoir des particules (59) attirées et rassemblées par l'aimant (40).

7. Outil médical (20) selon la revendication précédente, dans lequel le réservoir (50) présente une ouverture (54) d'un côté de l'outil médical (20), laquelle est opposée à une zone d'action (29) du dispositif d'enlèvement (24, 26, 28) de l'outil médical (20).

8. Outil médical (20) selon l'une quelconque des revendications précédentes, dans lequel au moins une surface polaire (42, 44) de l'aimant (40) est disposée au niveau d'un côté de l'outil médical (20) opposé à une zone d'action (29) de l'outil médical (20).

9. Outil médical (20) selon l'une quelconque des revendications précédentes, dans lequel le dispositif d'enlèvement (24, 26, 28) présente un matériau magnétique ou magnétisable.

10. Outil médical (20) selon l'une quelconque des revendications précédentes, dans lequel l'outil d'enlèvement (20) présente deux arêtes de coupe (24, 28) qui sont réalisées et disposées de manière à être déplacées l'une devant l'autre pour enlever des tissus.

11. Instrument médical (10) comprenant un outil médical (20) selon l'une quelconque des revendications précédentes.
